# FASCICULE DE BREVET EUROPEEN

(11) **EP 4 058 114 B1**
(45) Date de publication et mention de la délivrance du brevet: **06.03.2024**
(21) Numéro de dépôt: 20817465.6
(22) Date de dépôt: 10.11.2020
(51) Int. Cl.: A61M 11/00, A61M 5/28, A61M 15/00, A61M 15/08, B05B 11/02

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 12.11.2019 FR 1912629
(43) Date de publication de la demande: 21.09.2022
(73) Titulaire: APTAR France SAS, 27110 Le Neubourg (FR)
(72) Inventeur: BAILLET, Matthieu, 76000 ROUEN (FR); ESPINOZA, Diego, 76350 OISSEL (FR)
(74) Mandataire: CAPRI
(86) Numéro de dépôt international: PCT/FR2020/052054
(87) Numéro de publication internationale: WO 2021/094683

(56) Documents cités:
- WO-A1-2014/147329
- WO-A1-2016/001601
- WO-A1-2019/008260
- US-A- 5 944 222

## Description

La présente invention concerne un dispositif de distribution nasale de produit fluide.

Plus particulièrement, la présente invention concerne un dispositif de distribution nasale de produit fluide pour distribuer un produit fluide pharmaceutique à un utilisateur par l'intermédiaire d'une ou plusieurs pulvérisation(s) nasale(s).

Un inconvénient des dispositifs de distribution nasale de produit fluide existants concerne leur compatibilité de la tête de distribution avec des morphologies de nez différentes, notamment chez les enfants.

Par ailleurs, dans ce type de dispositif nasal, le spray est généralement réalisé au moyen d'un insert disposée dans la tête de distribution, en amont de l'orifice de distribution. Le moulage de ces pièces peut générer des problèmes, tels que des retassures. L'assemblage, notamment l'insertion, la tenue et l'étanchéité de l'insert dans la tête de distribution, peut aussi s'avérer complexe, avec des risques de dysfonctionnement du dispositif. En particulier, le profil de pulvérisation étant défini entre l'insert et la tête de distribution, les propriétés du spray peuvent varier d'un dispositif à l'autre selon les conditions d'assemblage.

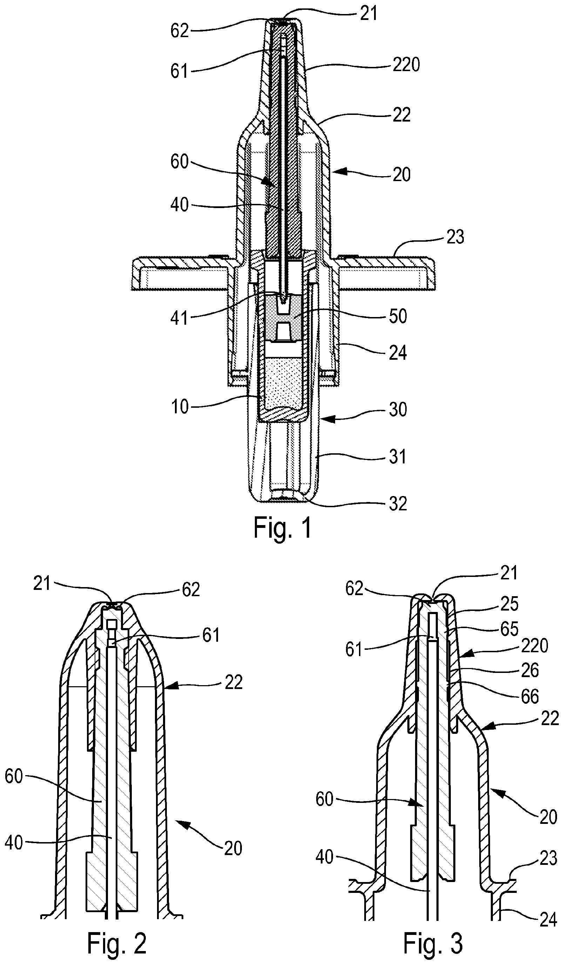

Le document WO2016001601 décrit un dispositif de l'état de la technique comportant une tête de distribution telle que représentée sur la figure 2.

La présente invention a pour but de fournir un dispositif de distribution de produit fluide qui ne reproduit pas les inconvénients susmentionnés.

Plus particulièrement, la présente invention a pour but de fournir un dispositif de distribution de produit fluide qui évite ou limite les risques de dysfonctionnement du dispositif et améliore sa robustesse.

La présente invention a également pour but de fournir un tel dispositif qui soit simple et peu coûteux à fabriquer et à assembler.

La présente invention a donc pour objet un dispositif de distribution de produit fluide, comportant un réservoir contenant du produit fluide, une tête de distribution pourvue d'un orifice de distribution, un bouchon-piston pour distribuer au moins une partie dudit produit fluide à travers ledit orifice de distribution, ledit réservoir étant, avant actionnement, obturé de manière étanche par ledit bouchon-piston, ledit dispositif comportant un insert creux inséré et fixé dans ladite tête de distribution, ledit insert supportant une canule pour percer ledit bouchon-piston et ainsi relier ledit réservoir audit orifice de distribution lors de l'actionnement, dans lequel:
- ladite tête de distribution comporte une partie d'extrémité proximale de diamètre réduit dont la longueur axiale est supérieure à 5 mm, avantageusement supérieure à 6 mm, dont le diamètre extérieur maximal est inférieur à 8 mm, avantageusement inférieur à 7 mm, et dont l'épaisseur de paroi est inférieure à 1,1 mm, avantageusement comprise entre 0,7 et 1,0 mm;
- ladite tête de distribution comporte une paroi latérale interne comportant une première section cylindrique proximale et une seconde section cylindrique distale de diamètre supérieur à celui de ladite première section cylindrique proximale, ledit insert comportant sur sa paroi externe une première projection radiale proximale s'étendant sur toute la périphérie, ladite première projection radiale proximale coopérant de manière serrante et étanche avec ladite première section cylindrique proximale, pour assurer la fixation étanche dudit insert dans ladite tête de distribution.

Avantageusement, ledit insert, à l'état assemblé, est en butée par sa surface frontale avec la paroi de fond de ladite tête de distribution, ladite butée formant le seul contact axial entre ledit insert et ladite tête de distribution.

Avantageusement, ladite partie d'extrémité proximale est légèrement conique, ladite partie d'extrémité proximale comportant niveau dudit orifice de distribution un diamètre extérieur inférieur à 6 mm, avantageusement inférieur à 5 mm.

Selon une première variante avantageuse, ladite partie d'extrémité proximale s'étend sur une distance axiale L1 d'au moins 5 mm, de préférence 6,15 mm, son diamètre extérieur D1 à son extrémité distale étant inférieur à 6 mm, notamment 5,7 mm, et son diamètre extérieur D1' à son extrémité proximale, au niveau dudit orifice de distribution, étant inférieur à 5 mm, notamment 4,6 mm.

Selon une seconde variante avantageuse, ladite partie d'extrémité proximale s'étend sur une distance axiale L2 d'au moins 10 mm, de préférence 12,5 mm, son diamètre extérieur D2 à son extrémité distale étant inférieur à 7 mm, notamment 6,6 mm, et son diamètre extérieur D2' à son extrémité proximale, au niveau dudit orifice de distribution, étant inférieur à 5 mm, notamment 4,6 mm.

Selon l'invention, ledit insert comporte sur sa paroi externe une seconde projection radiale distale, axialement séparée de ladite première projection radiale proximale, ladite seconde projection radiale proximale coopérant avec ladite seconde section cylindrique proximale pour assurer le guidage dudit insert lors de son insertion dans ladite tête de distribution, et le centrage en position assemblée.

Avantageusement, ladite première projection radiale proximale a une surface d'extrémité réalisant le serrage radial dont la dimension axiale est inférieure à 1 mm, avantageusement inférieure à 0,7 mm.

Avantageusement, ladite seconde projection radiale proximale a une surface d'extrémité dont la dimension axiale est inférieure à 1 mm, avantageusement inférieure à 0,6 mm.

Avantageusement, ledit insert comporte sur sa surface frontale un profil de pulvérisation disposé, à l'état assemblé, directement en amont dudit orifice de distribution.

Avantageusement, ledit insert définit un canal d'expulsion, au moins une ouverture radiale permettant au fluide de passer du canal d'expulsion audit profil de pulvérisation, ledit canal d'expulsion comportant une partie de canal proximale, au niveau de ladite ouverture radiale, une partie de canal centrale de diamètre supérieur à celui de ladite partie de canal proximale et reliée à cette dernière par un premier épaulement radial, et une partie de canal distale de diamètre supérieur à celui de ladite partie de canal centrale et reliée à cette dernière par un second épaulement radial.

Avantageusement, ladite canule est emmanchée à force dans ladite partie de canal centrale, et son extrémité proximale vient en butée contre ledit premier épaulement radial.

Avantageusement, ledit réservoir contient une seule dose de produit fluide, distribuée en un seul actionnement du dispositif.

En variante, ledit réservoir contient deux doses de produit fluide, distribuées en deux actionnements successifs du dispositif.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution de produit fluide selon un mode de réalisation avantageux, en position de repos avant actionnement,
La figure 2 est une vue de détail en section transversale d'une tête de distribution et d'un insert de l'art antérieur,
La figure 3 est une vue similaire à celle de la figure 2, montrant une tête de distribution et un insert selon un mode de réalisation avantageux de la présente invention,
La figure 4 est une vue de détail en section transversale de l'insert de la figure 3,
La figure 5 est une vue de côté de l'insert de la figure 3, et
Les figures 6a et 6b sont des vues de détail en section transversale de deux variantes de réalisation avantageuses de la tête de distribution.

Les termes "proximal" et "distal" se réfèrent à l'orifice de distribution. Les termes "axial" et "radial" se réfèrent à l'axe central longitudinal du dispositif. Les termes "amont" et "aval" se réfèrent à la direction d'écoulement du fluide lors de l'actionnement. Les termes "haut" et "bas" se réfèrent à la position droite du dispositif représentée sur la figure 1.

En référence aux figures 1 et 3 à 5, il est représenté un mode de réalisation avantageux de la présente invention.

Dans ce mode de réalisation, un réservoir 10 contenant du produit fluide à distribuer, typiquement un liquide, est disposé à l'intérieur d'un corps formant une tête de distribution 20. Cette tête de distribution 20 comporte un orifice de distribution 21 orienté axialement. Cet orifice de distribution 21 sert à distribuer une dose de produit fluide hors de ladite tête de distribution 20 lors de l'actionnement du dispositif par un utilisateur.

La tête de distribution 20 comporte un embout nasal allongé 22 comportant à son extrémité axiale proximale ledit orifice de distribution 21, ainsi qu'un corps latéral 24, relié audit embout nasal 22 par une bride radiale 23. La paroi latérale interne de l'embout nasal 22 comporte une première section cylindrique proximale 25 et une seconde section cylindrique distale 26 de diamètre supérieur à celui de la première section cylindrique proximale 25.

Un insert creux 60 est disposé dans la tête de distribution 20, en amont dudit orifice de distribution 21, ledit insert creux 60 définissant un canal d'expulsion 61, 61', 61" et, en coopération avec la paroi de fond de la tête de distribution 20, un profil de pulvérisation 62 directement en amont dudit orifice de distribution 21. Au moins une ouverture radiale 63, disposée à proximité de l'extrémité proximale de l'insert 60, permet au fluide de passer du canal d'expulsion 61 au profil de pulvérisation 62. Le canal d'expulsion comporte une partie de canal proximale 61, au niveau de ladite ouverture radiale 63. Le canal d'expulsion comporte aussi une partie de canal centrale 61' de diamètre supérieur à celui de la partie de canal proximale 61 et reliée à cette dernière par un premier épaulement radial 68. Enfin, le canal d'expulsion comporte également une partie de canal distale 61" de diamètre supérieur à celui de la partie de canal centrale 61' et reliée à cette dernière par un second épaulement radial 69. Sur sa paroi externe, l'insert 60 comporte une première projection radiale proximale 65 s'étendant sur toute la périphérie, ainsi qu'une seconde projection radiale distale 66, qui peut également s'étendre sur toute la périphérie, et qui est axialement séparée de ladite première projection radiale proximale 65.

Une canule creuse 40 est insérée et fixée dans ledit canal d'expulsion 61, 61', 61" ladite canule 40 comportant à son extrémité distale une pointe de perçage 41. Avantageusement, la canule 40 est emmanchée à force dans la partie de canal centrale 61', et son extrémité proximale vient en butée contre ledit premier épaulement radial 68. Ainsi, l'insertion de la canule 40 dans l'insert 60 est facilitée, le premier épaulement radial 68 définissant sa position d'assemblage, et l'emmanchement à force étant limitée à la partie de canal centrale 61'.

Le réservoir 10 est dans les exemples des figures formé par un corps creux et borgne, comportant une seule ouverture fermée par un bouchon-piston 50 et contenant une seule dose de produit fluide à distribuer un actionnement unique du dispositif.

Lors de l'actionnement, le réservoir 10 est déplacé axialement vers le haut par rapport à la tête de distribution 20 et donc par rapport à la canule 40. Le bouchon-piston 50 est ainsi poussé contre la pointe 41 de la canule 40 pour être percé, ce qui permet l'expulsion de tout ou partie du produit fluide contenu dans le réservoir 10.

Il est à noter que la présente invention peut aussi s'adapter à des dispositifs du type bidose ou multidoses, dans lequel le réservoir contient au moins deux doses de produit fluide à distribuer en au moins deux actionnements successifs du dispositif.

Des moyens d'actionnement 30 sont prévus pour permettre l'actionnement du dispositif.

Dans le mode de réalisation de la figure 1, ces moyens d'actionnement 30 comportent un corps d'actionnement 31 mobile par rapport à la tête de distribution 20, ledit corps d'actionnement 31 coopérant avec ledit réservoir 10 pour le déplacer axialement par rapport à la tête de distribution 20, en direction de l'orifice de distribution 21.

Le fonctionnement du dispositif va être détaillé ci-après.

De manière classique, l'utilisateur place deux doigts sur la bride radiale 23 formée sur la tête de distribution 20, et appuie avec le pouce sur le fond axial distal 32 dudit corps d'actionnement 31. Lors d'un tel actionnement, le réservoir 10 est donc poussé axialement en direction de l'orifice de distribution 21, de sorte que la canule 40 perce le bouchon-piston 50. Le contenu du réservoir 10 est alors relié à l'orifice de distribution 21 et l'appui de l'utilisateur sur le corps d'actionnement 31 déplace le bouchon-piston 50 dans le réservoir 10 pour assurer la distribution du produit fluide.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon et du côté distal par un second bouchon, le volume défini entre lesdits deux bouchons contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second bouchon à l'intérieur du réservoir. Or, le produit fluide étant incompressible, ce déplacement du second bouchon va donc déplacer le premier bouchon en direction de la canule, qui est fixe. Le premier bouchon va donc être percé par la canule et le contenu du réservoir va être distribué vers l'orifice de distribution, avec le second bouchon qui agit alors en tant que piston.

Comme évoqué précédemment, l'invention peut aussi s'appliquer à un dispositif du type bidose. Dans ce cas, le contenu du réservoir serait distribué en deux actionnements successifs. Le document WO2014147329 décrit un exemple de dispositif bidose.

Selon l'invention, la tête de distribution 20 et l'insert 60 ont des formes et dimensions particulières, qui améliorent le moulage et l'assemblage de ces pièces, ainsi que la fiabilité de fonctionnement du dispositif.

L'embout nasal 22 de la tête de distribution 20 comporte une partie d'extrémité proximale 220 de diamètre réduit. Cette partie d'extrémité proximale 220 est avantageusement légèrement conique. Elle a une longueur axiale supérieure à 5 mm, avantageusement supérieure à 6 mm. Elle comporte un diamètre extérieur maximal inférieur à 8 mm, avantageusement inférieur à 7 mm. Au niveau de l'orifice de distribution 21, ladite partie d'extrémité proximale 220 comporte avantageusement un diamètre extérieur inférieur à 6 mm, avantageusement inférieur à 5 mm.

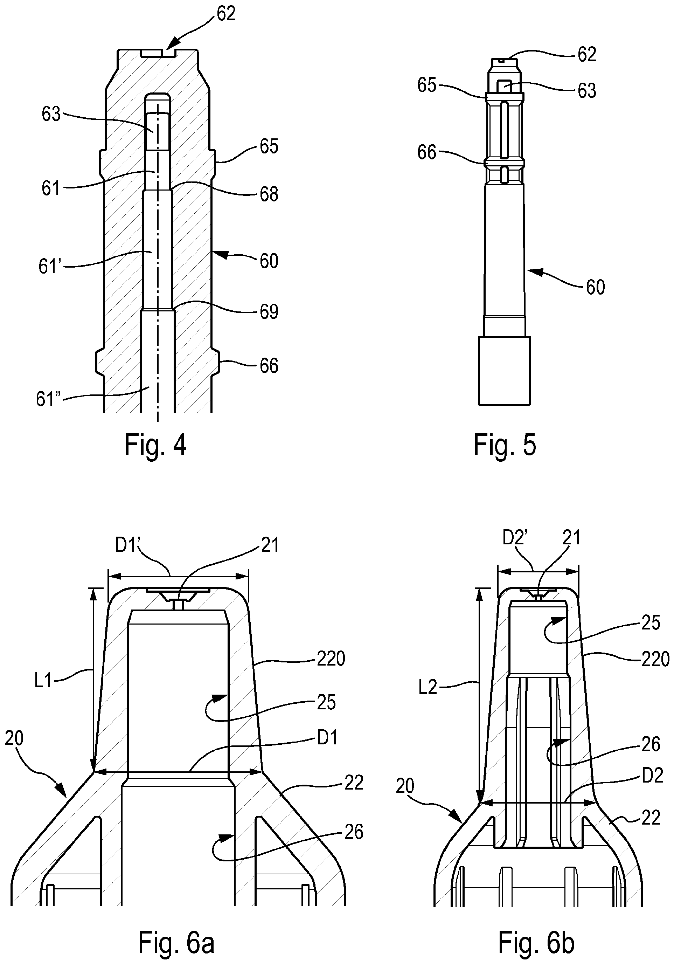

Dans la variante de la figure 6a, ladite partie d'extrémité proximale 220 s'étend sur une distance axiale L1 d'au moins 5 mm, de préférence 6,15 mm. Son diamètre extérieur D1 à l'extrémité distale est inférieur à 6 mm, de préférence 5,7 mm. Son diamètre extérieur D1' à l'extrémité proximale, au niveau de l'orifice de distribution 21, est inférieur à 5 mm, de préférence 4,6 mm.

Dans la variante de la figure 6b, ladite partie d'extrémité proximale 220 s'étend sur une distance axiale L2 d'au moins 10 mm, de préférence 12,5 mm. Son diamètre extérieur D2 à l'extrémité distale est inférieur à 7 mm, de préférence 6,6 mm. Son diamètre extérieur D2' à l'extrémité proximale, au niveau de l'orifice de distribution 21, est inférieur à 5 mm, de préférence 4,6 mm.

L'épaisseur de la paroi de ladite partie d'extrémité 220 est inférieure à 1,1 mm, avantageusement comprise entre 0,7 et 1,0 mm. Cette mise en oeuvre améliore le moulage de la tête de distribution 20, en réduisant notamment les risques de déformation liés à des surépaisseurs et en réduisant les risques de retassures.

Lorsque l'insert 60 est assemblé dans la tête de distribution 20, il est inséré dans celle-ci, jusqu'à ce que le profil de pulvérisation 62 disposé sur la surface frontale de l'insert vienne en butée contre la paroi de fond de la tête de distribution 20. Il s'agit du seul contact axial entre l'insert 60 et la tête de distribution. Il n'y a donc plus de double contact axial, comme dans le dispositif de l'art antérieur représenté sur la figure 2, qui était susceptible, notamment en raison des tolérances de fabrication de l'insert et de la tête de distribution, de générer des profils de sprays différents entre différents dispositifs. Avec la présente invention et son contact axial unique au niveau du profil de pulvérisation, on garantit que ce dernier présente toujours la même géométrie, avec par conséquent toujours les mêmes propriétés de spray.

En position assemblée, la première projection radiale proximale 65 coopère de manière serrante et étanche avec la première section cylindrique proximale 25 de l'embout nasal 22, pour assurer la fixation étanche de l'insert 60 dans la tête de distribution 20. La seconde projection radiale proximale 66 coopère avec la seconde section cylindrique proximale 26 de l'embout nasal 22. Cette coopération assure le guidage de l'insert 60 lors de son insertion dans la tête de distribution 20, et le centrage en position assemblée, et elle n'est donc ni étanche ni serrante.

La première projection radiale proximale 65 a une surface d'extrémité réalisant le serrage radial avec la première section cylindrique proximale 25. La dimension axiale de cette première projection radiale proximale 65 est inférieure à 1 mm, avantageusement inférieure à 0,7 mm, de préférence 0,63 mm. Ainsi, la surface serrante étant fortement réduite, l'assemblage de l'insert 60 dans la tête de distribution est facilité, nécessitant une force d'insertion inférieure par rapport au dispositif de l'art antérieur de la figure 2, dans lequel la surface serrante entre l'insert et la tête de distribution est typiquement supérieure à 5 mm. Les risques d'endommager l'insert lors de son assemblage sont donc réduits.

La seconde projection radiale distale 66 a une surface d'extrémité dont la dimension axiale est inférieure à 1 mm, avantageusement inférieure à 0,6 mm, de préférence 0,53 mm. Ainsi, même en cas de contact entre ladite seconde projection radiale distale 66 et la seconde section cylindrique distale 26 lors de l'assemblage, ce contact sur une surface de faible dimension n'altère pas le procédé d'assemblage.

La présente invention fournit donc un dispositif pour lequel la fabrication et l'assemblage de la tête de distribution et de son insert sont simplifiés, plus fiable et moins coûteux, rendant le dispositif plus robuste et garantissant des performances reproductibles.

Bien entendu, d'autres variantes de réalisation sont également envisageables, sans sortir du cadre de la présente invention, tel que défini par les revendications annexées.

## Revendications

1. Dispositif de distribution de produit fluide, comportant un réservoir (10) contenant du produit fluide, une tête de distribution (20) pourvue d'un orifice de distribution (21), un bouchon-piston (50) pour distribuer au moins une partie dudit produit fluide à travers ledit orifice de distribution (21), ledit réservoir (10) étant, avant actionnement, obturé de manière étanche par ledit bouchon-piston (50), ledit dispositif comportant un insert (60) creux inséré et fixé dans ladite tête de distribution (20), ledit insert (60) supportant une canule (40) pour percer ledit bouchon-piston (50) et ainsi relier ledit réservoir (10) audit orifice de distribution (21) lors de l'actionnement,
- ladite tête de distribution (20) comportant une partie d'extrémité proximale (220) de diamètre réduit dont la longueur axiale est supérieure à 5 mm, avantageusement supérieure à 6 mm, dont le diamètre extérieur maximal est inférieur à 8 mm, avantageusement inférieur à 7 mm, et dont l'épaisseur de paroi est inférieure à 1,1 mm, avantageusement comprise entre 0,7 et 1,0 mm;
- ladite tête de distribution (20) comportant une paroi latérale interne comportant une première section cylindrique proximale (25) et une seconde section cylindrique distale (26) de diamètre supérieur à celui de ladite première section cylindrique proximale (25), ledit insert (60) comportant sur sa paroi externe une première projection radiale proximale (65) s'étendant sur toute la périphérie, ladite première projection radiale proximale (65) coopérant de manière serrante et étanche avec ladite première section cylindrique proximale (25), pour assurer la fixation étanche dudit insert (60) dans ladite tête de distribution (20) ;
- ledit insert (60) comportant sur sa paroi externe une seconde projection radiale distale (66), axialement séparée de ladite première projection radiale proximale (65), ladite seconde projection radiale distale (66) coopérant avec ladite seconde section cylindrique distale (26) pour assurer le guidage dudit insert (60) lors de son insertion dans ladite tête de distribution (20), et le centrage en position assemblée.

2. Dispositif selon la revendication 1, dans lequel ledit insert (60), à l'état assemblé, est en butée par sa surface frontale avec la paroi de fond de ladite tête de distribution (20), ladite butée formant le seul contact axial entre ledit insert (60) et ladite tête de distribution (20).

3. Dispositif selon la revendication 1 ou 2, dans lequel ladite partie d'extrémité proximale (220) est légèrement conique, ladite partie d'extrémité proximale (220) comportant niveau dudit orifice de distribution (21) un diamètre extérieur inférieur à 6 mm, avantageusement inférieur à 5 mm.

4. Dispositif selon la revendication 3, dans lequel ladite partie d'extrémité proximale (220) s'étend sur une distance axiale (L1) d'au moins 5 mm, de préférence 6,15 mm, son diamètre extérieur (D1) à son extrémité distale étant inférieur à 6 mm, notamment 5,7 mm, et son diamètre extérieur (D1') à son extrémité proximale, au niveau dudit orifice de distribution (21), étant inférieur à 5 mm, notamment 4,6 mm.

5. Dispositif selon la revendication 3, dans lequel ladite partie d'extrémité proximale (220) s'étend sur une distance axiale (L2) d'au moins 10 mm, de préférence 12,5 mm, son diamètre extérieur (D2) à son extrémité distale étant inférieur à 7 mm, notamment 6,6 mm, et son diamètre extérieur (D2') à son extrémité proximale, au niveau dudit orifice de distribution (21), étant inférieur à 5 mm, notamment 4,6 mm.

6. Dispositif selon l'une quelconque des revendications précédentes, dans lequel ladite seconde projection radiale distale (66) a une surface d'extrémité dont la dimension axiale est inférieure à 1 mm, avantageusement inférieure à 0,6 mm.

7. Dispositif selon l'une quelconque des revendications précédentes, dans lequel ladite première projection radiale proximale (65) a une surface d'extrémité réalisant le serrage radial dont la dimension axiale est inférieure à 1 mm, avantageusement inférieure à 0,7 mm.

8. Dispositif selon l'une quelconque des revendications précédentes, dans lequel ledit insert (60) comporte sur sa surface frontale un profil de pulvérisation (62) disposé, à l'état assemblé, directement en amont dudit orifice de distribution (21).

9. Dispositif selon la revendication 8, dans lequel ledit insert (60) définit un canal d'expulsion (61, 61', 61"), au moins une ouverture radiale (63) permettant au fluide de passer du canal d'expulsion audit profil de pulvérisation (62), ledit canal d'expulsion comportant une partie de canal proximale (61), au niveau de ladite ouverture radiale (63), une partie de canal centrale (61 ') de diamètre supérieur à celui de ladite partie de canal proximale (61) et reliée à cette dernière par un premier épaulement radial (68), et une partie de canal distale (61 ") de diamètre supérieur à celui de ladite partie de canal centrale (61') et reliée à cette dernière par un second épaulement radial (69).

10. Dispositif selon la revendication 9, dans lequel ladite canule (40) est emmanchée à force dans ladite partie de canal centrale (61'), et son extrémité proximale vient en butée contre ledit premier épaulement radial (68).

11. Dispositif selon l'une quelconque des revendications précédentes, dans lequel ledit réservoir (10) contient une seule dose de produit fluide, distribuée en un seul actionnement du dispositif.

12. Dispositif selon l'une quelconque des revendications 1 à 10, dans lequel ledit réservoir (10) contient deux doses de produit fluide, distribuées en deux actionnements successifs du dispositif.

## Patentansprüche

1. Vorrichtung zur Ausgabe eines Fluidprodukts, aufweisend einen Vorratsbehälter (10), der Fluidprodukt enthält, einen Ausgabekopf (20), der mit einer Ausgabeöffnung (21) versehen ist, einen Kolbenstopfen (50) zur Ausgabe wenigstens eines Teils des Fluidprodukts durch die Ausgabeöffnung (21), wobei der Vorratsbehälter (10) vor Betätigung vom Kolbenstopfen (50) dicht verschlossen ist, wobei die Vorrichtung einen hohlen Einsatz (60) aufweist, der in den Ausgabekopf (20) eingefügt und darin befestigt ist, wobei der Einsatz (60) eine Kanüle (40) trägt, um den Kolbenstopfen (50) zu durchbohren und so bei der Betätigung den Vorratsbehälter (10) mit der Ausgabeöffnung (21) zu verbinden,
- wobei der Ausgabekopf (20) einen proximalen Endabschnitt (220) verringerten Durchmessers, dessen axiale Länge größer als 5 mm, vorteilhafterweise größer als 6 mm ist, dessen maximaler Außendurchmesser kleiner als 8 mm, vorteilhafterweise kleiner als 7 mm ist und dessen Wanddicke kleiner als 1,1 mm ist, vorteilhafterweise zwischen 0,7 und 1,0 mm beträgt;
- wobei der Ausgabekopf (20) eine innere Seitenwand aufweist, die einen ersten, proximalen zylindrischen Abschnitt (25) und einen zweiten, distalen zylindrischen Abschnitt (26) mit größerem Durchmesser als jener des ersten, proximalen zylindrischen Abschnitts (25) aufweist, wobei der Einsatz (60) an seiner Außenwand einen ersten, proximalen Radialvorsprung (65) aufweist, der sich über den gesamten Umfang erstreckt, wobei der erste, proximale Radialvorsprung (65) klemmend und dicht mit dem ersten, proximalen zylindrischen Abschnitt (25) zusammenwirkt, um die dichte Befestigung des Einsatzes (60) im Ausgabekopf (20) zu gewährleisten;
- wobei der Einsatz (60) an seiner Außenwand einen zweiten, distalen Radialvorsprung (66) aufweist, der axial vom ersten, proximalen Radialvorsprung (65) getrennt ist, wobei der zweite, distale Radialvorsprung (66) mit dem zweiten, distalen zylindrischen Abschnitt (26) zusammenwirkt, um die Führung des Einsatzes (60) bei dessen Einfügung in den Ausgabekopf (20) und die Zentrierung in Zusammenbauposition zu gewährleisten.

2. Vorrichtung nach Anspruch 1, wobei der Einsatz (60) im zusammengesetzten Zustand durch seine Frontfläche an der Bodenwand des Ausgabekopfes (20) anliegt, wobei die Anlage den einzigen axialen Kontakt zwischen dem Einsatz (60) und dem Ausgabekopf (20) bildet.

3. Vorrichtung nach Anspruch 1 oder 2, wobei der proximale Endabschnitt (220) leicht kegelförmig ist, wobei der proximale Endabschnitt (220) im Bereich der Ausgabeöffnung (21) einen Außendurchmesser kleiner als 6 mm, vorteilhafterweise kleiner als 5 mm aufweist.

4. Vorrichtung nach Anspruch 3, wobei sich der proximale Endabschnitt (220) über eine axiale Strecke (L1) von wenigstens 5 mm, vorzugsweise 6,15 mm erstreckt, wobei sein Außendurchmesser (D1) an seinem distalen Ende kleiner als 6 mm, insbesondere 5,7 mm ist, und sein Außendurchmesser (D1') an seinem proximalen Ende im Bereich der Ausgabeöffnung (21) kleiner als 5 mm, insbesondere 4,6 mm ist.

5. Vorrichtung nach Anspruch 3, wobei sich der proximale Endabschnitt (220) über eine axiale Strecke (L2) von wenigstens 10 mm, vorzugsweise 12,5 mm erstreckt, wobei sein Außendurchmesser (D2) an seinem distalen Ende kleiner als 7 mm, insbesondere 6,6 mm ist, und sein Außendurchmesser (D2') an seinem proximalen Ende im Bereich der Ausgabeöffnung (21) kleiner als 5 mm, insbesondere 4,6 mm ist.

6. Vorrichtung nach einem der vorhergehenden Ansprüche, wobei der zweite, distale Radialvorsprung (66) eine Endfläche aufweist, deren axiale Abmessung kleiner als 1 mm, vorteilhafterweise kleiner als 0,6 mm ist.

7. Vorrichtung nach einem der vorhergehenden Ansprüche, wobei der erste, proximale Radialvorsprung (65) eine Endfläche aufweist, welche die radiale Klemmung durchführt, deren axiale Abmessung kleiner als 1 mm, vorteilhafterweise kleiner als 0,7 mm ist.

8. Vorrichtung nach einem der vorhergehenden Ansprüche, wobei der Einsatz (60) auf seiner Frontfläche ein Zerstäubungsprofil (62) aufweist, das im zusammengesetzten Zustand direkt stromauf der Ausgabeöffnung (21) angeordnet ist.

9. Vorrichtung nach Anspruch 8, wobei der Einsatz (60) einen Ausstoßkanal (61, 61', 61") definiert, wobei es wenigstens eine radiale Öffnung (63) dem Fluid ermöglicht, von dem Ausstoßkanal zum Zerstäubungsprofil (62) zu gelangen, wobei der Ausstoßkanal einen proximalen Kanalabschnitt (61) im Bereich der radialen Öffnung (63), einen mittleren Kanalabschnitt (61') mit einem größeren Durchmesser als jener des proximalen Kanalabschnitts (61) und der mit letzterem durch einen ersten Radialabsatz (68) verbunden ist, und einen distalen Kanalabschnitt (61") mit einem größeren Durchmesser als jener des mittleren Kanalabschnitts (61') und der mit letzterem durch einen zweiten Radialabsatz (69) verbunden ist, aufweist.

10. Vorrichtung nach Anspruch 9, wobei die Kanüle (40) in den mittleren Kanalabschnitt (61') eingepresst ist und ihr proximales Ende an dem ersten Radialabsatz (68) anliegt.

11. Vorrichtung nach einem der vorhergehenden Ansprüche, wobei der Vorratsbehälter (10) eine einzelne Fluidproduktdosis enthält, die mit einer einzelnen Betätigung der Vorrichtung ausgegeben wird.

12. Vorrichtung nach einem der Ansprüche 1 bis 10, wobei der Vorratsbehälter (10) zwei Fluidproduktdosen enthält, die mit zwei aufeinanderfolgenden Betätigungen der Vorrichtung ausgegeben werden.

## Claims

1. A fluid product dispenser device comprising a reservoir (10) containing fluid product, a dispenser head (20) that is provided with a dispenser orifice (21), a stopper/piston (50) for dispensing at least a portion of said fluid product through said dispenser orifice (21), said reservoir (10) being closed in sealed manner by said stopper/piston (50) before actuation, said device including a hollow insert (60) that is inserted and fixed in said dispenser head (20), said insert (60) supporting a cannula (40) for piercing said stopper/piston (50) and thus connecting said reservoir (10) to said dispenser orifice (21) during actuation,
- said dispenser head (20) including a proximal end portion (220) of reduced diameter the axial length of which is greater than 5 mm, advantageously greater than 6 mm, the maximum outside diameter of which is less than 8 mm, advantageously less than 7 mm, and the wall thickness of which is less than 1.1 mm, advantageously between 0.7 and 1.0 mm,
- said dispenser head (20) comprising an inner side wall comprising a first proximal cylindrical section (25) and a second distal cylindrical section (26) of diameter greater than that of said first proximal cylindrical section (25), said insert (60) comprising on its outer wall a first proximal radial projection (65) extending over the entire periphery, said first proximal radial projection (65) cooperating in a tight and sealed manner with said first proximal cylindrical section (25), in order to ensure the sealed fixing of said insert (60) in said dispenser head (20),
- said insert (60) including, on its outer wall, a second distal radial projection (66), axially separated from said first proximal radial projection (65), said second distal radial projection (66) co-operating with said second distal cylindrical section (26) to ensure the guidance of said insert (60) when it is inserted into said dispenser head (20), and the centering in the assembled position.

2. The device according to claim 1, wherein said insert (60), in the assembled state, abuts with its front surface against the bottom wall of said dispensing head
(20), said abutment forming the only axial contact between said insert (60) and said dispensing head (20).

3. The device according to claim 1 or claim 2, wherein said proximal end portion (220) is slightly conical, said proximal end portion (220) having at said dispenser orifice (21) an outside diameter of less than 6 mm, advantageously less than 5 mm.

4. The device according to claim 3, wherein said proximal end portion (220) extends over an axial distance (L1) of at least 5 mm, preferably 6.15 mm, its outer diameter (D1) at its distal end being less than 6 mm, in particular 5.7 mm, and its outer diameter (D1') at its proximal end, at said dispenser orifice (21), being less than 5 mm, in particular 4.6 mm.

5. The device according to claim 3, wherein said proximal end portion (220) extends over an axial distance (L2) of at least 5 mm, preferably 12.5 mm, its outer diameter (D2) at its distal end being less than 7 mm, in particular 6.6 mm, and its outer diameter (D2') at its proximal end, at said dispenser orifice (21), being less than 5 mm, in particular 4.6 mm.

6. The device according to any preceding claim, wherein said second distal radial projection (66) has an end surface the axial dimension of which is less than 1 mm, advantageously less than 0.6 mm.

7. The device according to any preceding claim, wherein said first proximal radial projection (65) has a radially clamping end surface the axial dimension of which is less than 1 mm, advantageously less than 0.7 mm.

8. The device according to any one of the preceding claims, wherein the said insert (60) has on its front surface a spray profile (62) arranged, in the assembled state, directly upstream of the said dispenser orifice (21).

9. The device according to claim 8, wherein said insert (60) defines an expulsion channel (61, 61', 61"), at least one radial opening (63) allowing the fluid to pass from the expulsion channel to said spray profile (62), said expulsion channel comprising a proximal channel portion (61), at said radial opening (63), a central channel portion (61') of diameter greater than that of said proximal channel portion (61) and connected to the latter by a first radial shoulder (68), and a distal channel portion (61") of diameter greater than that of said central channel portion (61') and connected to the latter by a second radial shoulder (69).

10. The device according to claim 9, wherein said cannula (40) is force-fitted into said central channel portion (61'), and its proximal end abuts said first radial shoulder (68).

11. The device according to any preceding claim, wherein said reservoir (10) contains a single dose of fluid dispensed in a single actuation of the device.

12. The device according to any one of claims 1 to 10, wherein said reservoir (10) contains two doses of fluid dispensed in two successive actuations of the device.
